# EUROPEAN PATENT APPLICATION

(11) **EP 1 925 625 A2**
(43) Date of publication of application: **28.05.2008**
(21) Application number: 07021912.6
(22) Date of filing: 02.10.2003
(51) Int. Cl.: C07K 7/06, C07K 7/08, C07K 14/00, G01N 33/50

(54) **Method for screening modulators of mitochondrial functionning and new modulators obtained**

(30) Priority: 02.10.2002 US 415092 P; 23.05.2003 US 472725 P
(62) Divisional of application: 03758082.6
(71) Applicant: Theraptosis S.A., 75015 Paris (FR)
(72) Inventor: Jacotot, Etienne, 75015 Paris (FR); Lecoeur, Hervé, 75014 Paris (FR); Rebouillat, Dominique, 75015 Paris (FR)
(74) Representative: Peaucelle, Chantal

(57) **Abstract**

The invention relates to a method for screening modulators of mitochondrial function comprising adding a compound to be tested in a purified, isolated mitochondria preparation and simultaneously using fluorimetric analysis of mitochondrial morphology, and especially real-time fluorimetric analysis, combining analysis of morphometric parameters (SSC/FSC parameters) with analysis of membrane integrity by dye fluorescence.

Application to the obtention of peptides which induce mitochondrial membrane permeabilization.

SEQUENCE LISTING

<110>  THERAPTOSIS S.A.

<120>  METHOD FOR SCREENING MODULATORS OF MITOCHONDRIAL FUCTIONNING AND
       NEW MODULATORS OBTAINED

<130>  60815-2909

<150>  US 60/472,725
<151>  2003-05-23

<150>  PCT/EP03/12056
<151>  2003-10-03

<150>  US 60/415/092
<151>  2002-10-02

<160>  57

<170>  PatentIn version 3.3

<210>  1
<211>  16
<212>  PRT
<213>  Artificial sequence

<220>
<223>  Synthetic peptide

<400>  1

Ala Thr Leu Ser Ala Leu Leu Ala Ala Leu Arg Arg Ile Gln Arg Ala
1               5                   10                  15


<210>  2
<211>  27
<212>  PRT
<213>  Artificial sequence

<220>
<223>  Synthetic peptide

<400>  2

Arg Lys Lys Arg Arg Gln Arg Arg Arg Gly Gly Ala Thr Leu Ser Ala
1               5                   10                  15


Leu Leu Ala Ala Leu Arg Arg Ile Gln Arg Ala
            20                  25


<210>  3
<211>  40
<212>  PRT
<213>  Artificial sequence

<220>
<223>  Synthetic peptide

<400>  3

Arg Lys Lys Arg Arg Gln Arg Arg Arg Cys Gly Gly Leu Glu Thr Arg
1               5                   10                  15

Thr Glu Thr Trp Met Ser Ser Glu Gly Ala Trp Lys Gln Ile Gln Lys
            20                  25                  30

Val Glu Thr Trp Ala Leu Arg His
        35                  40

<210>  4
<211>  40
<212>  PRT
<213>  Artificial sequence

<220>
<223>  Synthetic peptide

<400>  4

Arg Lys Lys Arg Arg Gln Arg Arg Arg Cys Gly Gly Leu Ala Asn Lys
1               5                   10                  15

Lys Gly Ala Trp Leu Asp Ser Thr Lys Ala Thr Arg Tyr Leu Val Lys
            20                  25                  30

Thr Glu Ser Trp Ile Leu Arg Asn
        35                  40

<210>  5
<211>  28
<212>  PRT
<213>  Artificial sequence

<220>
<223>  Synthetic peptide

<220>
<221>  DISULFID
<222>  (3)..(10)

<400>  5

Gly Gly Cys Arg Gly Asp Met Phe Gly Cys Gly Gly Leu Leu Phe Ile
1               5                   10                  15

His Phe Arg Ile Gly Ser Arg His Ser Arg Ile Gly
            20                  25

<210>  6
<211>  9
<212>  PRT
<213>  Artificial sequence

<220>
<223>  Synthetic peptide

<400>  6

Arg Ile Glu Ile Trp Ile Leu Arg His
1               5

<210>  7

<211>  9
<212>  PRT
<213>  Artificial sequence

<220>
<223>  Synthetic peptide

<400>  7

Arg Ile Ala Ile Trp Ile Leu Arg His
1               5


<210>  8
<211>  20
<212>  PRT
<213>  Artificial sequence

<220>
<223>  Synthetic peptide

<400>  8

Arg Lys Lys Arg Arg Gln Arg Arg Arg Gly Gly Arg Ile Glu Ile Trp
1               5                   10                  15


Ile Leu Arg His
            20


<210>  9
<211>  20
<212>  PRT
<213>  Artificial sequence

<220>
<223>  Synthetic peptide

<400>  9

Arg Lys Lys Arg Arg Gln Arg Arg Arg Gly Gly Arg Ile Ala Ile Trp
1               5                   10                  15


Ile Leu Arg His
            20


<210>  10
<211>  28
<212>  PRT
<213>  Artificial sequence

<220>
<223>  Synthetic peptide

<400>  10

Glu His Trp Ser Tyr Trp Leu Arg Pro Gly Gly Gly Leu Leu Phe Ile
1               5                   10                  15


His Phe Arg Ile Gly Cys Arg His Ser Arg Ile Gly
            20                  25


<210>  11

<211>  31
<212>  PRT
<213>  Artificial sequence

<220>
<223>  Synthetic peptide

<400>  11

Glu His Trp Ser Tyr Trp Leu Arg Pro Gly Gly Gly Gly Gly Ser Leu
1               5                   10                  15

Leu Phe Ile His Phe Arg Ile Gly Cys Arg His Ser Arg Ile Gly
            20                  25                  30

<210>  12
<211>  27
<212>  PRT
<213>  Artificial sequence

<220>
<223>  Synthetic peptide

<400>  12

Arg Lys Lys Arg Arg Gln Arg Arg Arg Gly Gly Gly Ala Trp Lys His
1               5                   10                  15

Ala Gln Arg Ile Glu Ile Trp Ile Leu Arg His
            20                  25

<210>  13
<211>  27
<212>  PRT
<213>  Artificial sequence

<220>
<223>  Synthetic peptide

<400>  13

Arg Lys Lys Arg Arg Gln Arg Arg Arg Gly Gly Gly Ala Trp Lys His
1               5                   10                  15

Ala Gln Arg Ile Glu Thr Trp Ile Leu Arg His
            20                  25

<210>  14
<211>  27
<212>  PRT
<213>  Artificial sequence

<220>
<223>  Synthetic peptide

<400>  14

Arg Lys Lys Arg Arg Gln Arg Arg Arg Gly Gly Gly Ala Trp Lys His
1               5                   10                  15

Ala Gln Arg Val Glu Ser Trp Ile Leu Arg Asn

20                  25


<210>  15
<211>  27
<212>  PRT
<213>  Artificial sequence

<220>
<223>  Synthetic peptide

<400>  15

Arg Lys Lys Arg Arg Gln Arg Arg Arg Gly Gly Gly Ala Trp Lys Arg
1               5                   10                  15


Ala Cys Arg Met Glu Thr Trp Ile Leu Arg His
            20                  25


<210>  16
<211>  27
<212>  PRT
<213>  Artificial sequence

<220>
<223>  Synthetic peptide

<400>  16

Arg Lys Lys Arg Arg Gln Arg Arg Arg Gly Gly Gly Ala Trp Lys Gln
1               5                   10                  15


Ile Gln Lys Val Glu Thr Trp Ala Leu Arg His
            20                  25


<210>  17
<211>  27
<212>  PRT
<213>  Artificial sequence

<220>
<223>  Synthetic peptide

<400>  17

Arg Lys Lys Arg Arg Gln Arg Arg Arg Gly Gly Gly Ala Trp Arg Gln
1               5                   10                  15


Val Glu Lys Val Glu Thr Trp Ala Leu Arg His
            20                  25


<210>  18
<211>  27
<212>  PRT
<213>  Artificial sequence

<220>
<223>  Synthetic peptide

<400>  18

Arg Lys Lys Arg Arg Gln Arg Arg Arg Gly Gly Gly Ala Trp Lys His

1                    5                        10                       15

Ala Gln Arg Ile Ala Ile Trp Ile Leu Arg His
                20                       25

<210>  19
<211>  15
<212>  PRT
<213>  Artificial sequence

<220>
<223>  synthetic peptide

<400>  19

Ala Trp Lys His Ala Gln Arg Ile Ala Ile Trp Ile Leu Arg His
1                   5                        10                       15

<210>  20
<211>  21
<212>  PRT
<213>  Artificial sequence

<220>
<223>  synthetic peptide

<220>
<221>  DISULFID
<222>  (3)..(10)

<400>  20

Gly Gly Cys Arg Gly Asp Met Phe Gly Cys Gly Gly Arg Ile Glu Ile
1                   5                        10                       15

Trp Ile Leu Arg His
                20

<210>  21
<211>  21
<212>  PRT
<213>  Artificial sequence

<220>
<223>  synthetic peptide

<220>
<221>  DISULFID
<222>  (3)..(10)

<400>  21

Gly Gly Cys Arg Gly Asp Met Phe Gly Cys Gly Gly Arg Ile Ala Ile
1                   5                        10                       15

Trp Ile Leu Arg His
                20

<210>  22

<211>  22
<212>  PRT
<213>  Artificial sequence

<220>
<223>  Synthetic peptide


<220>
<221>  DISULFID
<222>  (3)..(11)

<400>  22

Gly Gly Cys Gly Arg Gly Asp Ser Pro Gly Cys Gly Gly Arg Ile Glu
1               5                   10                  15


Ile Trp Ile Leu Arg His
            20


<210>  23
<211>  22
<212>  PRT
<213>  Artificial sequence

<220>
<223>  Synthetic peptide


<220>
<221>  DISULFID
<222>  (3)..(11)

<400>  23

Gly Gly Cys Gly Arg Gly Asp Ser Pro Gly Cys Gly Gly Arg Ile Ala
1               5                   10                  15


Ile Trp Ile Leu Arg His
            20


<210>  24
<211>  21
<212>  PRT
<213>  Artificial sequence

<220>
<223>  Synthetic peptide

<400>  24

Glu His Trp Ser Tyr Trp Leu Arg Pro Gly Gly Gly Arg Ile Glu Ile
1               5                   10                  15


Trp Ile Leu Arg His
            20


<210>  25
<211>  21
<212>  PRT
<213>  Artificial sequence

<220>
<223>  Synthetic peptide

<400>  25

Glu His Trp Ser Tyr Trp Leu Arg Pro Gly Gly Gly Arg Ile Ala Ile
1               5                   10                  15

Trp Ile Leu Arg His
            20

<210>  26
<211>  24
<212>  PRT
<213>  Artificial sequence

<220>
<223>  Synthetic peptide

<400>  26

Glu His Trp Ser Tyr Trp Leu Arg Pro Gly Gly Gly Gly Gly Ser Arg
1               5                   10                  15

Ile Glu Ile Trp Ile Leu Arg His
            20

<210>  27
<211>  24
<212>  PRT
<213>  Artificial sequence

<220>
<223>  Synthetic peptide

<400>  27

Glu His Trp Ser Tyr Trp Leu Arg Pro Gly Gly Gly Gly Gly Ser Arg
1               5                   10                  15

Ile Ala Ile Trp Ile Leu Arg His
            20

<210>  28
<211>  31
<212>  PRT
<213>  Artificial sequence

<220>
<223>  Synthetic peptide

<400>  28

Glu His Trp Ser Tyr Trp Leu Arg Pro Gly Gly Gly Gly Gly Ser Gly
1               5                   10                  15

Ala Trp Lys His Ala Gln Arg Ile Glu Ile Trp Ile Leu Arg His
            20                  25                  30

<210>  29

<211>  31
<212>  PRT
<213>  Artificial sequence

<220>
<223>  synthetic peptide

<400>  29

Glu His Trp Ser Tyr Trp Leu Arg Pro Gly Gly Gly Gly Gly Ser Gly
1               5                   10                  15

Ala Trp Lys His Ala Gln Arg Ile Ala Ile Trp Ile Leu Arg His
            20                  25                  30

<210>  30
<211>  28
<212>  PRT
<213>  Artificial sequence

<220>
<223>  synthetic peptide

<400>  30

Glu His Trp Ser Tyr Trp Leu Arg Pro Gly Gly Gly Leu Leu Phe Ile
1               5                   10                  15

His Phe Lys Ile Gly Cys Lys His Ser Lys Ile Gly
            20                  25

<210>  31
<211>  31
<212>  PRT
<213>  Artificial sequence

<220>
<223>  synthetic peptide

<400>  31

Glu His Trp Ser Tyr Trp Leu Arg Pro Gly Gly Gly Gly Gly Ser Leu
1               5                   10                  15

Leu Phe Ile His Phe Lys Ile Gly Cys Lys His Ser Lys Ile Gly
            20                  25                  30

<210>  32
<211>  28
<212>  PRT
<213>  Artificial sequence

<220>
<223>  synthetic peptide

<400>  32

Glu His Trp Ser Tyr Trp Leu Arg Pro Gly Gly Gly Leu Leu Phe Ile
1               5                   10                  15

His Phe Arg Ile Gly Ser Arg His Ser Arg Ile Gly

20                  25

<210>  33
<211>  31
<212>  PRT
<213>  Artificial sequence

<220>
<223>  synthetic peptide

<400>  33

Glu His Trp Ser Tyr Trp Leu Arg Pro Gly Gly Gly Gly Gly Ser Leu
1               5                   10                  15

Leu Phe Ile His Phe Arg Ile Gly Ser Arg His Ser Arg Ile Gly
            20                  25                  30

<210>  34
<211>  28
<212>  PRT
<213>  Artificial sequence

<220>
<223>  synthetic peptide

<400>  34

Glu His Trp Ser Tyr Trp Leu Arg Pro Gly Gly Gly Leu Leu Phe Ile
1               5                   10                  15

His Phe Lys Ile Gly Ser Lys His Ser Lys Ile Gly
            20                  25

<210>  35
<211>  31
<212>  PRT
<213>  Artificial sequence

<220>
<223>  synthetic peptide

<400>  35

Glu His Trp Ser Tyr Trp Leu Arg Pro Gly Gly Gly Gly Gly Ser Leu
1               5                   10                  15

Leu Phe Ile His Phe Lys Ile Gly Ser Lys His Ser Lys Ile Gly
            20                  25                  30

<210>  36
<211>  27
<212>  PRT
<213>  Artificial sequence

<220>
<223>  synthetic peptide

<400>  36

Arg Lys Lys Arg Arg Gln Arg Arg Arg Gly Gly Gly Ala Trp Lys His

1                   5                   10                  15

Ala Gln Arg Ile Glu Ile Trp Ile Leu Arg His
            20                  25

<210>  37
<211>  27
<212>  PRT
<213>  Artificial sequence

<220>
<223>  Synthetic peptide

<400>  37

Arg Lys Lys Arg Arg Gln Arg Arg Arg Gly Gly Gly Ala Trp Lys His
1               5                   10                  15

Ala Gln Arg Ile Glu Thr Trp Ile Leu Arg His
            20                  25

<210>  38
<211>  27
<212>  PRT
<213>  Artificial sequence

<220>
<223>  Synthetic peptide

<400>  38

Arg Lys Lys Arg Arg Gln Arg Arg Arg Gly Gly Gly Ala Trp Lys His
1               5                   10                  15

Ala Gln Arg Val Glu Ser Trp Ile Leu Arg Asn
            20                  25

<210>  39
<211>  27
<212>  PRT
<213>  Artificial sequence

<220>
<223>  Synthetic peptide

<400>  39

Arg Lys Lys Arg Arg Gln Arg Arg Arg Gly Gly Gly Ala Trp Lys Arg
1               5                   10                  15

Ala Cys Arg Met Glu Thr Trp Ile Leu Arg His
            20                  25

<210>  40
<211>  27
<212>  PRT
<213>  Artificial sequence

<220>
<223>  Synthetic peptide

<400>  40

Arg Lys Lys Arg Arg Gln Arg Arg Arg Gly Gly Gly Ala Trp Lys Gln
1               5                   10                  15

Ile Gln Lys Val Glu Thr Trp Ala Leu Arg His
            20                  25

<210>  41
<211>  27
<212>  PRT
<213>  Artificial sequence

<220>
<223>  Synthetic peptide

<400>  41

Arg Lys Lys Arg Arg Gln Arg Arg Arg Gly Gly Gly Ala Trp Arg Gln
1               5                   10                  15

Val Glu Lys Val Glu Thr Trp Ala Leu Arg His
            20                  25

<210>  42
<211>  21
<212>  PRT
<213>  Artificial sequence

<220>
<223>  Synthetic peptide

<400>  42

Glu His Trp Ser Tyr Trp Leu Arg Pro Gly Gly Gly Arg Ile Glu Ile
1               5                   10                  15

Trp Ile Leu Arg His
            20

<210>  43
<211>  21
<212>  PRT
<213>  Artificial sequence

<220>
<223>  Synthetic peptide

<400>  43

Glu His Trp Ser Tyr Trp Leu Arg Pro Gly Gly Gly Arg Ile Ala Ile
1               5                   10                  15

Trp Ile Leu Arg His
            20

<210>  44
<211>  24
<212>  PRT

<213>  Artificial sequence

<220>
<223>  Synthetic peptide

<400>  44

Glu His Trp Ser Tyr Trp Leu Arg Pro Gly Gly Gly Gly Gly Ser Arg
1               5                   10                  15

Ile Glu Ile Trp Ile Leu Arg His
            20

<210>  45
<211>  24
<212>  PRT
<213>  Artificial sequence

<220>
<223>  Synthetic peptide

<400>  45

Glu His Trp Ser Tyr Trp Leu Arg Pro Gly Gly Gly Gly Gly Ser Arg
1               5                   10                  15

Ile Ala Ile Trp Ile Leu Arg His
            20

<210>  46
<211>  31
<212>  PRT
<213>  Artificial sequence

<220>
<223>  Synthetic peptide

<400>  46

Glu His Trp Ser Tyr Trp Leu Arg Pro Gly Gly Gly Gly Gly Ser Gly
1               5                   10                  15

Ala Trp Lys His Ala Gln Arg Ile Glu Ile Trp Ile Leu Arg His
            20                  25                  30

<210>  47
<211>  31
<212>  PRT
<213>  Artificial sequence

<220>
<223>  Synthetic peptide

<400>  47

Glu His Trp Ser Tyr Trp Leu Arg Pro Gly Gly Gly Gly Gly Ser Gly
1               5                   10                  15

Ala Trp Lys His Ala Gln Arg Ile Ala Ile Trp Ile Leu Arg His
            20                  25                  30

<210>  48
<211>  28
<212>  PRT
<213>  Artificial sequence

<220>
<223>  Synthetic peptide

<400>  48

Glu His Trp Ser Tyr Trp Leu Arg Pro Gly Gly Gly Leu Leu Phe Ile
1               5                   10                  15

His Phe Lys Ile Gly Cys Lys His Ser Lys Ile Gly
            20                  25

<210>  49
<211>  31
<212>  PRT
<213>  Artificial sequence

<220>
<223>  Synthetic peptide

<400>  49

Glu His Trp Ser Tyr Trp Leu Arg Pro Gly Gly Gly Gly Gly Ser Leu
1               5                   10                  15

Leu Phe Ile His Phe Lys Ile Gly Cys Lys His Ser Lys Ile Gly
            20                  25                  30

<210>  50
<211>  28
<212>  PRT
<213>  Artificial sequence

<220>
<223>  Synthetic peptide

<400>  50

Glu His Trp Ser Tyr Trp Leu Arg Pro Gly Gly Gly Leu Leu Phe Ile
1               5                   10                  15

His Phe Arg Ile Gly Ser Arg His Ser Arg Ile Gly
            20                  25

<210>  51
<211>  31
<212>  PRT
<213>  Artificial sequence

<220>
<223>  Synthetic peptide

<400>  51

Glu His Trp Ser Tyr Trp Leu Arg Pro Gly Gly Gly Gly Gly Ser Leu
1               5                   10                  15

Leu Phe Ile His Phe Arg Ile Gly Ser Arg His Ser Arg Ile Gly
            20                  25                  30

<210>  52
<211>  28
<212>  PRT
<213>  Artificial sequence

<220>
<223>  Synthetic peptide

<400>  52

Glu His Trp Ser Tyr Trp Leu Arg Pro Gly Gly Gly Leu Leu Phe Ile
1               5                   10                  15

His Phe Lys Ile Gly Ser Lys His Ser Lys Ile Gly
            20                  25

<210>  53
<211>  31
<212>  PRT
<213>  Artificial sequence

<220>
<223>  Synthetic peptide

<400>  53

Glu His Trp Ser Tyr Trp Leu Arg Pro Gly Gly Gly Gly Gly Ser Leu
1               5                   10                  15

Leu Phe Ile His Phe Lys Ile Gly Ser Lys His Ser Lys Ile Gly
            20                  25                  30

<210>  54
<211>  21
<212>  PRT
<213>  Artificial sequence

<220>
<223>  Synthetic peptide

<220>
<221>  DISULFID
<222>  (3)..(10)

<400>  54

Gly Gly Cys Arg Gly Asp Met Phe Gly Cys Gly Gly Arg Ile Glu Ile
1               5                   10                  15

Trp Ile Leu Arg His
            20

<210>  55
<211>  21
<212>  PRT
<213>  Artificial sequence

<220>
<223>  Synthetic peptide

<220>
<221>  DISULFID
<222>  (3)..(10)

<400>  55

Gly Gly Cys Arg Gly Asp Met Phe Gly Cys Gly Gly Arg Ile Ala Ile
1               5                   10                  15

Trp Ile Leu Arg His
            20

<210>  56
<211>  22
<212>  PRT
<213>  Artificial sequence

<220>
<223>  Synthetic peptide

<220>
<221>  DISULFID
<222>  (3)..(11)

<400>  56

Gly Gly Cys Gly Arg Gly Asp Ser Pro Gly Cys Gly Gly Arg Ile Glu
1               5                   10                  15

Ile Trp Ile Leu Arg His
            20

<210>  57
<211>  22
<212>  PRT
<213>  Artificial sequence

<220>
<223>  Synthetic peptide

<220>
<221>  DISULFID
<222>  (3)..(11)

<400>  57

Gly Gly Cys Gly Arg Gly Asp Ser Pro Gly Cys Gly Gly Arg Ile Ala
1               5                   10                  15

Ile Trp Ile Leu Arg His
            20

## Description

The invention relates to a method for screening modulators of mitochondrial functioning and to the new modulators obtained.

During the last ten years, an intensive international research revealed the key role of mitochondria in apoptosis and other related forms of programmed cell death. Although mitochondrion is not the only organelle able to regulate apoptotic cellular demise, a large array of experimental evidences suggest that mitochondria is a critical component of the "central executioner" of apoptosis. As a result, the study of the regulation of mitochondrial function *in vitro,* using isolated mitochondria, are of a great interest both to decipher cell death mechanisms and to identify new therapeutic drugs.

Mitochondrial metabolism and permeability state of mitochondrial membranes are essential checkpoints for cell life and death decisions, and indeed, during mammalian programmed cell death, mitochondria are subjected to different structural and functional modifications. Experimentally, agents that block respiratory chain trigger bio-emergent catastrophe and molecules able to modify mitochondrial membrane permeability are efficient regulators of cell death including necrotic, apoptotic and autophagic types of cell death. During programmed cell death, also called apoptosis, mitochondrial modifications usually include: matrix swelling, outer membrane permeabilization (OMP) to intermembrane apoptosis effectors (such as cytochrome c, apoptosis-inducing factor, endoG, and Smac/Diablo), as well as dissipation of the inner transmembrane potential (Δψm). The exact hierarchy of these events is still a matter of debate and may depend both on cell type and cell death pathways. A well-known phenomenon often responsible for this mitochondrial membrane permeabilization (MMP) is the Mitochondrial Permeability Transition (MPT). MPT can lead to a rapid mitochondrial swelling (i.e. an increase in mitochondrial size and a decrease in the organelles' granularity), to the rupture of the outer membrane, and can induce a dissipation of the mitochondrial transmembrane potential (Δψm).

Pro- and anti-apoptotic members of the Bcl-2 family regulate inner and outer mitochondrial membrane permeabilization (MMP) through interactions with the adenine nucleotide translocator (ANT; in the inner membrane, IM), the voltage-dependent anion channel (VDAC; in the outer membrane, OM), and/or through autonomous channel-forming activities. ANT and VDAC are major components of the permeability transition pore (PTP), a polyprotein structure organized at sites at which the two mitochondrial membranes are apposed. The PTP is a non-specific pore, permeant to any molecule of < 1.5 kDa, which opens in the inner mitochondrial membrane under conditions of oxidative stress, high Ca2+ or low ATP concentrations. Opening of the PTP causes massive swelling of mitochondria, rupture of the outer membrane and release of intermembrane components that induce apoptosis. In addition mitochondria become depolarized causing inhibition of oxidative phosphorylation and stimulation of ATP hydrolysis. Pore opening is inhibited by cyclosporin A or derivatives of this compound that bind to mitochondrial cyclophilin (peptidyl-prolyl-cis-trans-isomerase).

The inventors have found that morphological and Δψm changes of isolated mitochondria can be quantified simultaneously in real-time by FCM (flow cytometry) analysis on a per-mitochondrion basis. Using this approach, they have developed a method for screening modulators of mitochondrial functioning and have obtained a new family of peptidic inducers of MMP.

The progressive mitochondrial swelling is detected by the significant increase in forward scatter (FSC) and the decrease in the side scatter (SSC) of the organelles. Both observations are significantly correlated with spectrophotometric absorbance data. Variations in Δψm are followed after loading with the JC-1 probe. This cationic lipophilic dye forms J-aggregates in highly energized mitochondria (emitting red fluorescence), whereas only present in monomers in the matrix of low Δψm organelles (associated with green fluorescence). The drop of the Δψm is followed by the progressive decrease in JC-1 red fluorescence and in the JC-1 red / green fluorescence ratio, both resulting from the dissociation of J-aggregates.

The invention thus relates to a method for screening modulators of mitochondrial function by real-time flow cytometry analysis, and is characterized in the Examples 1 to 3.

Example 1 describes the characterization of mitochondrial preparation by FCM: although purified mouse liver mitochondria are small organelles (1-2 µm), they are detectable by flow cytometry using the Δψm -insensitive dye MitoTracker^{®} Green, for a purity reaching 95%. Moreover, their functionality can be assessed using the Δψm -sensitive dye JC-1: most of these organelles are energetically competent since more than 90% have an elevated Δψm).

Example 2 shows how swelling quantification can be performed on isolated mitochondria, and the advantages of real- time analysis versus fixed time flow cytometry. Indeed, fixed time and real time FCM allow following changes induced by the PTP opener calcium. Real time allows integral calculus to estimate the velocity of these morphological changes.

However, for slow changes in mitochondrial morphology such as the protonophore carbonyl cyanide *meta-*chlorophenylhydrazone (CCCP), real-time is advantageous as it allows to follow a low but significant granularity (SSC) decrease without detectable size (FSC) changes. Indeed, using real-time fluorimetric follow-up of the mean SSC signal and the mean forward scatter (FSC) signal, it appears that CCCP induces a reduction of SSC that cannot be detected by classical spectrophotometric absorbance analysis.

When PTP-independent mitochondrial swelling is induced by the ion channel forming antibiotic peptide alamethicin, a simultaneous FSC increase and SSC decrease is also observed. Interestingly, with this molecule, SSC variations were less rapid than FSC modifications. Thus, real time analysis allows o better define different PTP modifiers.

Example 3 demonstrates how the area of real-time fluorimetric analysis can be extended to ΔΨm. Real time analysis allows to characterize mitochondria integrity and to differentiate various PTP-dependant and PTP-independent MMP inducers. Indeed, it allows a rapid identification of the mechanistic aspects of Calcium, alamethicin and CCCP, testing these drugs alone or in association with inhibitors such as Bongkretic acid, ruthenium red and Cyclosporin A. As an example of the utility of the method, these analyses indicate that at least in these experimental conditions, ΔΨm loss does not precede Ca2+ induced mitochondrial internal structure remodeling. Thus simultaneous flow cytometry real-time recording of mitochondrial size/structure and ΔΨm state may be used as a tool to define the hierarchy of MMP events.

Simultaneous detection of SSC and JC1-orange is thus a rapid and acute method to screen and quantify effects of MMP-inhibitors.

As shown in the examples above, this real-time approach is useful for characterization of the mitochondrial membrane permeabilization (MMP) induced by various molecules including peptides and small molecules. Said real-time approach is also useful to study the effect of different inhibitors of the respiratory chain (malonate, antimycin A, KCN, oligomycin, or the protonophore uncoupler CCCP) on MMP.

The invention thus relates to a method for screening modulators of mitochondrial functioning comprising adding a compound to be tested in a purified, isolated mitochondria preparation and simultaneously using a real-time fluorimetric analysis of mitochondrial morphology combining analysis of morphometric parameters (.SSC/FSC parameters) with analysis of membrane intergrity by dye fluorescence.

The analysis of the membrane integrity is performed using the JC-1 dye to characterize mitochondrial transmembrane potential Δψm and study any change of the mitochondrial membrane permeability.

The mechanism of mitochondrial membrane permeabilization is further characterized using pharmacological inducers or modulators of mitochondrial permeability such as Calcium, CCCp, Alamethicin, Bongkretic acid, ruthenium red, cyclostorin A, DIDS.

Such a method enables to study for example the impact of Bcl-2 family members and respiratory inhibitors on PTP-related MMP, and is also useful for example to identify and design new agents inducing mitochondrial permeability changes, alone or in combination, said compounds being for example chemicals, peptides, biological extracts from human or non human origin (e.g. from mammalian, animal, bacterial, yeast, or plant, either natural or created by genetic engineering).

The invention also relates to the use of said method for designing new agents aiming at modulating MMP.

The invention alos concerns a method such as above defined comprising the diagnosis or characterization of mitochondrial function in patients with diseases, and especially genetic diseases or metabolic diseases.

Said above defined methods are also useful to define agents able to cure defects thus revealed, any follow patient evolution.

In a specific embodiment, using the above-mentioned real time FCM analysis of mitochondria for screening, the inventors defined mitochondrially active peptides.

The invention thus relates to synthetic peptides which induce mitochondrial membrane permeabilization (MMP) when added to purified, isolated, mitochondria, said peptide being composed of L or D aminoacids, protected at the C terminus by amide or similar protection, and in N-ter by acyl, alkyl, biotinyl groups, or other modifications.

The invention also relates to said peptides and their derivative as containing simple addition, deletions, or mutations without altering the mitochondrial function.

Peptides according to the invention have the following sequences :

| | |
|---|---|
| ATLSALLAALRRIQRA | (SEQ ID N°1) |
| RKKRRQRRRGGATLSALLAALRRIQRA | (SEQ ID N°2) |
| RKKRRQRRRCGGLETRTETWMSSEGAWKQIQKVETWALRH | (SEQ ID N°3) |
| RKKRRQRRRCGGLANKKGAWLDSTKATRYLVKTESWILRN | (SEQ ID N°4) |
| GG*CRGDMFG*CGGLLFIHFRIGSRHSRIG | (SEQ ID N°5) |
| RIEIWILRH | (SEQ ID N°6) |
| RIAIWILRH | (SEQ ID N°7) |
| RKKRRQRRRGGRIEIWILRH | (SEQ ID N°8) |
| RKKRRQRRRGGRIAIWILRH | (SEQ ID N°9) |
| EHWSYWLRPGGGLLFIHFRIGCRHSRIG | (SEQ ID N°10) |
| EHWSYWLRPGGGGGSLLFIHFRIGCRHSRIG | (SEQ ID N°11) |

and their derivatives.

In this list, asterisk indicate disulfide bridge between cysteins.

In sequences ID 10 and 11, the W6 residue can advantageously be replaced by the D-aminoacid _{D}W.

Said peptides were shown to induce mitochondrial changes and MMP, and have the capacity to induce apoptosis in cultured cells, and thus have thepotential to be used to treat some human diseases.

Said mitochondrial properties of these sequences are shown in Example 4, demonstrating the ability of the technique to detect mitochondrial permeability inducers.

The inventors observed, during the above testing, a set of structural features highly indicative of a potency to act on mitochondrial permeability. This structural particularity can be used for the identification or design of a broad range of peptides modulating MMP.

The invention relates to any peptide containing at least 8 amino acids and until 50 amino acids having the following features:
- at least a part of the peptide structure is an amphipathic alpha helix,
- 2, 3 or 4 amino acids are positively charged (lysine [K] or arginine [R]) and are located on the same side of the helix,
- when the helix is projected using helical wheel representation, the R and/or K amino acids form a cluster (see figure 15),
- when added to purified, isolated, mitochondria they induce changes (ultrastructural or membrane permeability).

Examples of peptides presenting these characteristics and having mitochondrial activity are listed below:

| | |
|---|---|
| RKKRRQRRRGGGAWKHAQRIEIWILRH | (SEQ ID N°12) |
| RKKRRQRRRGGGAWKHAQRIETWILRH | (SEQ ID N°13) |
| RKKRRQRRRGGGAWKHAQRVESWILRN | (SEQ ID N°14) |
| RKKRRQRRRGGGAWKRACRMETWILRH | (SEQ ID N°15) |
| RKKRRQRRRGGGAWKQIQKVETWALRH | (SEQ ID N°16) |
| RKKRRQRRRGGGAWRQVEKVETWALRH | (SEQ ID.N°17) |
| RKKRRQRRRGGGAWKHAQRIAIWILRH | (SEQ ID N°18) |
| AWKHAQRIAIWILRH | (SEQ ID N°19) |
| GG*CRGDMFG*CGGRIEIWILRH | (SEQ ID N°20) |
| GG*CRGDMFG*CGGRIAIWILRH | (SEQ ID N°21) |
| GG*CGRGDSPG*CGGRIEIWILRH | (SEQ ID N°22) |
| GG*CGRGOSPG*CGGRIAIW1LRH | (SEQ ID N°23) |

Where *C = cysteine engaged in cycling disulfide bridge

| | |
|---|---|
| EHWSYWLRPGGGRIEIWILRH | (SEQ ID N°24) |
| EHWSYWLRPGGGRIAIWILRH | (SEQ ID N°25) |
| EHWSYWLRPGGGGGSRIEIWILRH | (SEQ ID N°26) |
| EHWSYWLRPGGGGGSRIAIWILRH | (SEQ ID N°27) |
| EHWSYWLRPGGGGGSGAWKHAQRIEIWILRH | (SEQ ID N°28) |
| EHWSYWLRPGGGGGSGAWKHAQRIAIWILRH | (SEQ ID N°29) |
| EHWSYWLRPGGGLLFIHFKIGCKHSKIG | (SEQ ID N°30) |
| EHWSYWLRPGGGGGSLLFIHFKIGCKHSKIG | (SEQ ID N°31) |
| EHWSYWLRPGGGLLFIHFRIGSRHSRIG | (SEQ ID N°32) |
| EHWSYWLRPGGGGGSLLFIHFRIGSRHSRIG | (SEQ ID N°33) |
| EHWSYWLRPGGGLLFIHFKIGSKHSKIG | (SEQ ID N°34) |
| EHWSYWLRPGGGGGSLLFIHFKIGSKHSKIG | (SEQ ID N°35) |

In which the W6 residue can advantageously be replaced by the D-aminoacid _{d}w.

Said peptides advantageously comprise in the C-terminal position a stabilizing group such as an amide group and a marker or linking group, such as the biotinyl group. They can also include L- or D- aminoacids retro-inverso, reduced peptidic backbone, or being translated into pseudo peptides.

The invention also relates to said peptides bound to a peptidic delivery system, and optionally comprising a linker between said peptides and delivery system. This peptidic delivery system could advantageously being an extra cellular or intracellular targeting sequence, an antibody or a fragment thereof (ScFv). The linker can be a sequence allowing the peptide to adopt an helical structure independently of the peptidic delivery system, and can be formed by 2 to 6 aminoacids such as alanine or glycine, or a disulfide bridge, or any such means as known by a man skilled in the art considering the state of the art knowledge. Said peptide could also be modified by insertions, deletions or mutations which conserve mitochondrial modulation potency.

Said peptide can be divided in families depending of the target cells for a possible therapeutic use:
Group 1: synthetic peptides that induce cell death of various cell types. Potency of these peptides to induce cell apoptosis is shown in Example 5.

| | |
|---|---|
| RKKRRQRRRGGRIEIWILRH | (SEQ ID N°8) |
| RKKRRQRRRGGRIAIWILRH | (SEQ ID N°9) |
| RKKRRQRRRGGGAWKHAQRIEIWILRH | (SEQ ID N°36) |
| RKKRRQRRRGGGAWKHAQRIETWILRH | (SEQ ID N°37) |
| RKKRRQRRRGGGAWKHAQRVESWILRN | (SEQ ID N°38) |
| RKKRRQRRRGGGAWKRACRMETWILRH | (SEQ ID N°39) |

| | |
|---|---|
| RKKRRQRRRGGGAWKQIQKVETWALRH | (SEQ ID N°40) |
| RKKRRQRRRGGGAWRQVEKVETWALRH | (SEQ ID N°41) |
| RKKRRQRRRGGATLSALLAALRRIQRA | (SEQ ID N°2) |
| RKKRRQRRRCGGLETRTETWMSSEGAWKQIQKVETWALRH | (SEQ ID N°3) |
| RKKRRQRRRCGGLANKKGAWLDSTKATRYLVKTESWILRN | (SEQ ID N° 4) |

Group 2: synthetic peptides that induce cell death of adenocarcinoma cell lines: a demonstration of potency of these peptides on adenocarcinoma cells is shown in Example 6.

| | |
|---|---|
| EHWSYWLRPGGGRIEIWILRH | (SEQ ID N°42) |
| EHWSYWLRPGGGRIAIWILRH | (SEQ ID N°43) |
| EHWSYWLRPGGGGGSRIEIWILRH | (SEQ ID N°44) |
| EHWSYWLRPGGGGGSRIAIWILRH | (SEQ ID N°45) |
| EHWSYWLRPGGGGGSGAWKHAQRIEIWILRH | (SEQ ID N°46) |
| EHWSYWLRPGGGGGSGAWKHAQRIAIWILRH | (SEQ ID N°47) |
| EHWSYWLRPGGGLLFIHFRIGCRHSRIG | (SEQ ID N°10) |
| EHWSYWLRPGGGGGSLLFIHFRIGCRHSRIG | (SEQ ID N°11) |
| EHWSYWLRPGGGLLFIHFKIGCKHSKIG | (SEQ ID N°48) |
| EHWSYWLRPGGGGGSLLFIHFKIGCKHSKIG | (SEQ ID N°49) |
| EHWSYWLRPGGGLLFIHFRIGSRHSRIG | (SEQ ID N°50) |
| EHWSYWLRPGGGGGSLLFIHFRIGSRHSRIG | (SEQ ID N°51) |
| EHWSYWLRPGGGLLFIHFKIGSKHSKIG | (SEQ ID N°52) |
| EHWSYWLRPGGGGGSLLFIHFKIGSKHSKIG | (SEQ ID N°53) |

Group 3: synthetic peptides that induce cell death of human umbilical vein embryonic cells (HUVECs). Demonstration of activity is shown in Example 7.

| | |
|---|---|
| GG*CRGDMFG*CGGRIEIWILRH | (SEQ ID N°54) |
| GG*CRGDMFG*CGGRIAIWILRH | (SEQ ID N°55) |
| GG*CGRGDSPG*CGGRIEIWILRH | (SEQ ID N°56) |
| GG*CGRGDSPG*CGGRIAIWILRH | (SEQ ID N°57) |
| GG*CRGDMFG*CGGLLFIHFRIGSRHSRIG | (SEQ ID N°5) |

*C = cysteine engaged in cycling disulfide bridge

Thus, the invention provides means for having molecules acting positively or negatively on MPP. As shown by the inventors, above described peptides induce MMP: cell-permeable versions of these mitochondrio-active peptides are efficient inducers of Δψm loss and cell death by apoptosis. Therefore, said new « mito-» fluorometric approach can be a useful and reliable tool for the identification of new mitochondrio-toxic or mitochondrio-protective drugs.

In addition, by using screened molecules and chimeric molecules composed of the mitochondrio-toxic domain sequences and a peptidic delivery system to induce cell death of tumoral cell lines (especially adenocarcinoma), or HUVECs, the inventors demonstrate the application of these sequences as potential mitochondrio-toxic molecules useful for designing new therapeutics molecules.

Molecules acting positively on MPP are useful for treating for example cancers and autoimmune diseases, and molecules negatively acting on MPP will display a cytoprotective effect (neuroprotection, cardioprotection and hepatoprotection, for example against acute or chronic ischemia). Such drugs may also be useful to treat metabolic disorders such as obesity and diabetes. More particularly, the molecules of the invention are useful for treating pathologies correlated with MPP or other dysfunctions including cell death.

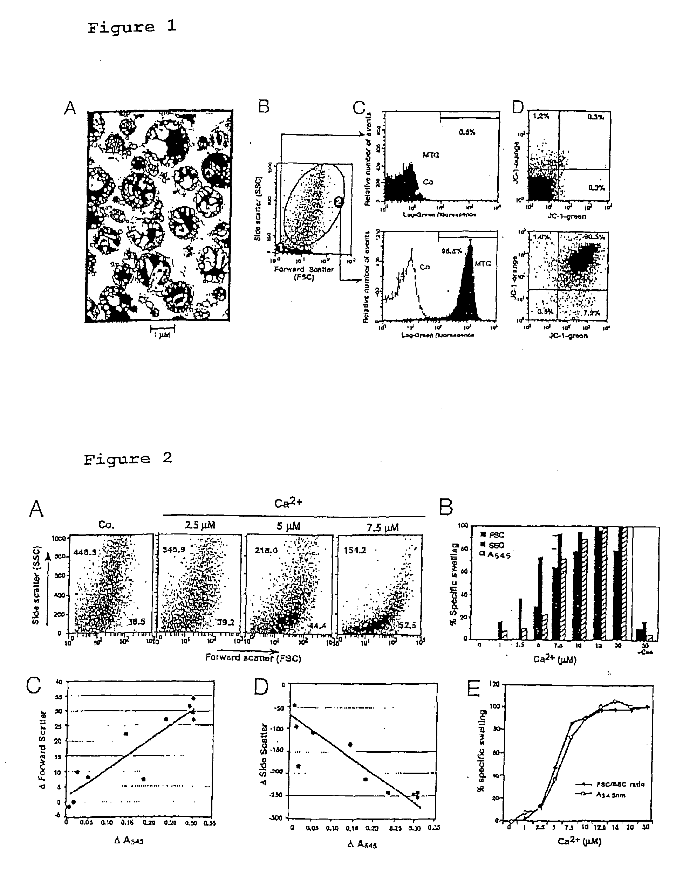

Other characteristics and advantages of the invention are given in the following, with reference to figures 1 to 13.

### Example 1: Mitochondrial analysis using flow cytometry

Purified mouse liver mitochondria are small organelles (1-2 µm); Fig. 1A), which are detectable by flow cytometry using the Δψm -insensitive dye MitoTracker^{®} Green. Indeed, correct gating on FSC/SSC parameter permits to select a population of events containing 95,5% of mitochondria (fig. 1B, C). FSC, which is highly correlated to cell size/volume (Salzman et al. 1990) permits to evaluate mitochondrial size, whereas the SSC parameter, which is related to granularity and refractive index, is a physical parameter correlated with mitochondrial internal structure. Most of these organelles are energetically competent since more than 90% have an elevated Δψm as revealed through labeling with the ΔΨm -sensitive dye JC-1 (Fig. 1D).

### Example 2: Mitochondrial swelling measurement: fixed time vs. real time FCM

Calcium (Ca²⁺) acts on mitochondria as a PTP activator (opener) to induce a dose-dependent SSC decrease and FSC increase (Fig. 2 A, B). This effect is highly correlated with dose-dependent loss of absorbance at 545 nm (classical swelling; Fig. 2 B, C, D). Interestingly, SSC decrease (internal structure modifications) appears to be the most sensitive parameters that can be measured after addition of low Ca²⁺ doses (Fig. 2B). Indeed SSC were detected at higher extent than classical swelling (Absorbance) until 7.5 µM (Fig. 2B). As expected these changes are reduced by pre-treatment of mitochondria with the PTP inhibitor (closer) cyclosporin A (CsA; Fig.2B). In addition, FSC/SSC ratio is highly correlated to spectrophotometric data (Fig. 2E). Altogether, those data indicates that flow cytometry could reliably evidence mitochondrial swelling by evaluating mitochondrial SSC (internal structure) and FSC (size).

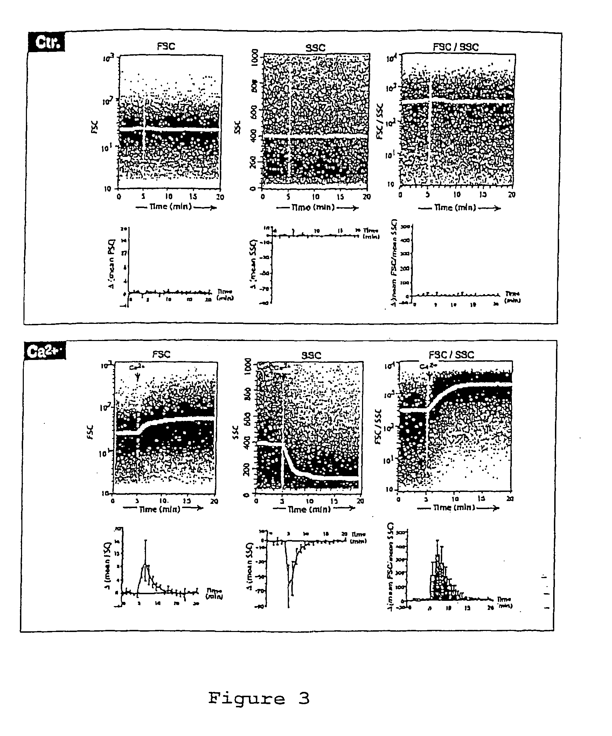

Real-time Flow cytometric recording of size and structure parameters can extend the potentialities of this approach. For this purpose, basal FSC and SSC characteristics of mitochondria are recorded for 20 minutes in the absence (Fig. 3; Ctr.) or presence of Ca²⁺ (Fig. 3; Ca²⁺) . No modifications are observed for the FSC, SSC and FSC/SSC ratio in control mitochondria. On the contrary, Ca²⁺ induces striking increase in FSC, FSC/SSC ratio, and a rapid decrease of mitochondrial structure (SSC) (Fig. 3; Ca²⁺). Then, integral calculus can be used to estimate the velocity of these morphological changes (Fig.3D). Interestingly, the velocity of structure modifications (SSC) after Ca²⁺ addition is maximal within the first minute of treatment, suggesting that the SSC parameter is a very sensitive sensor of swelling-related modifications.

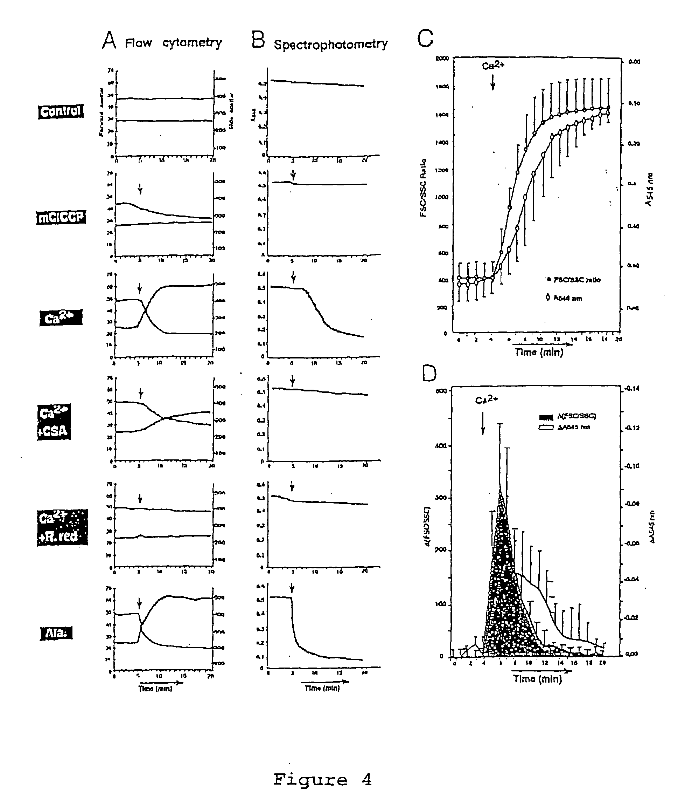

When treated with the protonophore carbonyl cyanide *meta-*chlorophenylhydrazone (CCCP), the mitochondrial population is subjected to a low but significant granularity (SSC) decrease without detectable size (FSC) changes. Indeed, using real-time fluorimetric follow-up of the mean SSC signal and the mean forward scatter (FSC) signal, it appears that CCCP induces a reduction of SSC (Fig. 4A) that cannot be detected by classical spectrophotometric absorbance analysis (Fig. 4B). Ca²⁺ induced PTP-opening is associated with a rapid and strong FSC/SSC ratio increase, which is the consequence of simultaneous FSC increase and SSC decrease. Moreover, CsA-mediated inhibition of Ca²⁺-induced changes can be correctly followed by real-time FSC-SSC analysis (Fig. 4). When PTP-independent mitochondrial swelling is induced by the ion channel forming antibiotic peptide alamethicin, a simultaneous FSC increase and SSC decrease is also observed. Interestingly, with this molecule, SSC variations were less rapid than FSC modifications. Alamethicin induced MMP is not inhibited by CsA pretreatment (not shown) and well correlated with spectrophotometric absorbance analysis (Fig.4 A, B). In addition, real-time fluorimetric follow-up of FSC/SSC ratio after alamethicin or Ca²⁺ treatment is sensitive and well correlated with classical spectrophotometric absorbance analysis (Fig. 4 C, D).

### Example 3: Mitochondrial function assessed by real time FCM

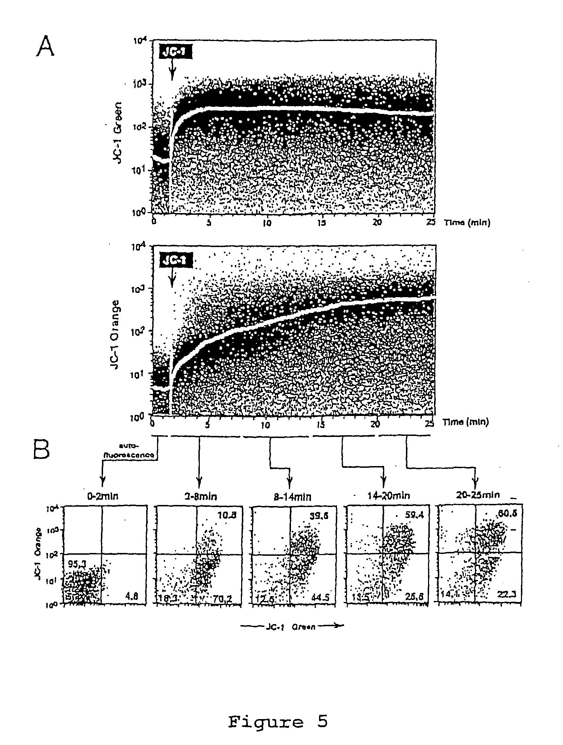

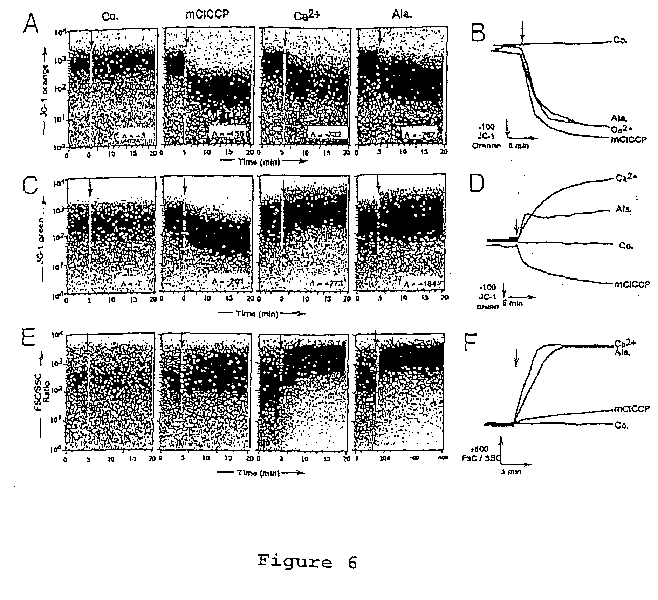

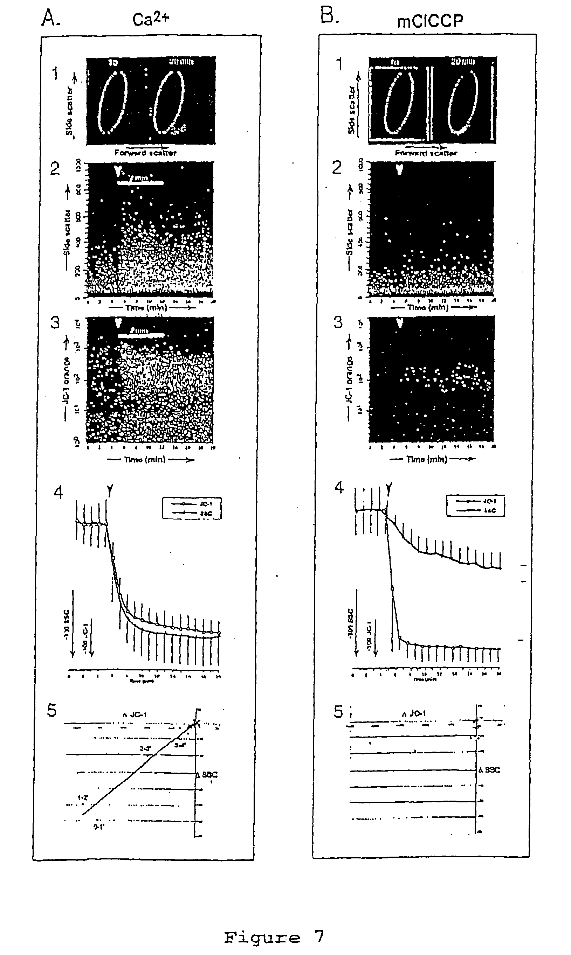

The area of real-time fluorimetric analysis can be extended to ΔΨm using the carbocyanine dye 5,5',6,6',-tetracholoro-1,1,3,3'-tetraethylbenzimidazolylcarbocyanine iodide (JC-1). When purified mitochondria are loaded with JC-1 (0,11 nanomoles JC-1 / □g protein), J-aggregate formation (orange-red fluorescence) increases with time to reach a maximum after 15-20 minutes (Fig. 5). ΔΨm suppression by CCCP addition induces a rapid decrease in JC-1 orange fluorescence and a slow decrease in monomeric JC-1 green fluorescence (Fig. 6). In contrast, PTP-dependant (induced by Ca²⁺) and PTP-independent (induced by alamethicin) mitochondrial membranes permeabilization (MMP) provoke both a decrease in JC-1 orange fluorescence and a significant increase in JC-1 green fluorescence. These phenomena most probably result from the dissociation of J-aggregates into JC-1 monomers. As expected, alamethicin and Ca²⁺ but not CCCP induce a rapid increase in mitochondrial FSC/SSC ratio (Fig. 6E). Simultaneous real-time analysis of both SSC (swelling) and JCl-orange (ΔΨm loss) decrease after Ca2+ treatment reveals a clear-cut correlation between these parameters. These analyses also indicate that at least in these experimental conditions, ΔΨm loss does not precede Ca2⁺ induced mitochondrial internal structure remodeling (Fig. 7). In contrast, CCCP treatment induced a rapid ΔΨm loss, which precede a very slow and low SSC reduction. Thus simultaneous flow cytometric real-time recording of mitochondrial size/structure and ΔΨm state may be used as a tool to define the hierarchy of MMP events.

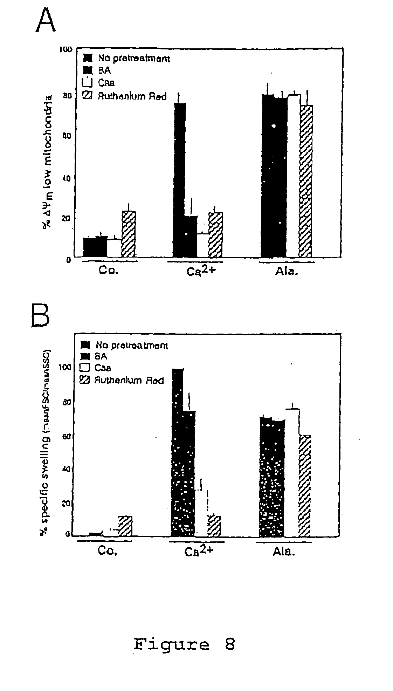

Simultaneous detection of SSC and JC1-orange is also a rapid method to screen and quantify effects of MMP-inhibitors. Accordingly, CsA the reference PTP-inhibitor completely abolish Ca²⁺ induced SSC decrease and ΔΨm loss (Fig. 8). Pre-treatment with bongkrekic acid (BA), an ANT ligand which affect ANT conformation, strongly reduces Ca²⁺ induced ΔΨm loss and partly affect SSC decrease. Another efficient inhibitor of Ca²⁺ induced MMP is ruthenium red which prevent accumulation of Ca²⁺ into mitochondria through the pharmacological inhibition of the Ca²⁺ uniporter. Ruthenium red inhibits 80% of Ca2+ induced ΔΨm loss and SSC decrease (Fig. 8). In contrast, alamethicin induced MMP is not affected by CsA, BA and ruthenium red.

### Example 4: Mitochondrial alterations induced by peptides

The peptides corresponding to

| | |
|---|---|
| ATLSALLAALRRIQRA | (SEQ ID N°1) |
| RKKRRQRRRGGATLSALLAALRRIQRA | (SEQ ID N°2) |
| RKKRRQRRRCGGLETRTETWMSSEGAWKQIQKVETWALRH | (SEQ ID N°3) |
| RKKRRQRRRCGGLANKKGAWLDSTKATRYLVKTESWILRN | (SEQ ID N°4) |
| GG*CRGDMFG*CGGLLFIHFRIGSRHSRIG | (SEQ ID N°5) |
| RIEIWILRH | (SEQ ID N°6) |
| RIAIWILRH | (SEQ ID N°7) |
| RKKRRQRRRGGRIEIWILRH | (SEQ ID N°8) |
| RKKRRQRRRGGRIAIWILRH | (SEQ ID N°9) |
| EHWSYWLRPGGGLLFIHFRIGCRHSRIG | (SEQ ID N°10) |
| EHWSYWLRPGGGGGSLLFIHFRIGCRHSRIG | (SEQ ID N°11) |

Were tested in the mitochondrial real time FCM analysis and for their swelling properties, at doses ranging from 1 to 20 µM. The results in terms of induction of mitochondrial changes are summarized below in Table 1 :

**Table 1: peptide induction of MMP in isolated mitochondria.**

| peptide | Significant FSC/ SSC increase | More than 50% ΔΨm loss | swelling |
|---|---|---|---|
| SEQ ID N°1 | + | + | + |
| SEQ ID N°2 | ND | ND | + |
| SEQ ID N°3 | + | + | + |
| SEQ ID N°4 | + | + | + |
| SEQ ID N°5 | + | + | + |
| SEQ ID N°6 | + | + | + |
| SEQ ID N°7 | + | + | + |
| SEQ ID N°8 | + | + | + |
| SEQ ID N°9 | ND | ND | + |
| SEQ ID N°10 | + | + | + |
| SEQ ID N°11 | ND | ND | + |

### Example 5: Induction of cellular apoptosis by MMP inducers

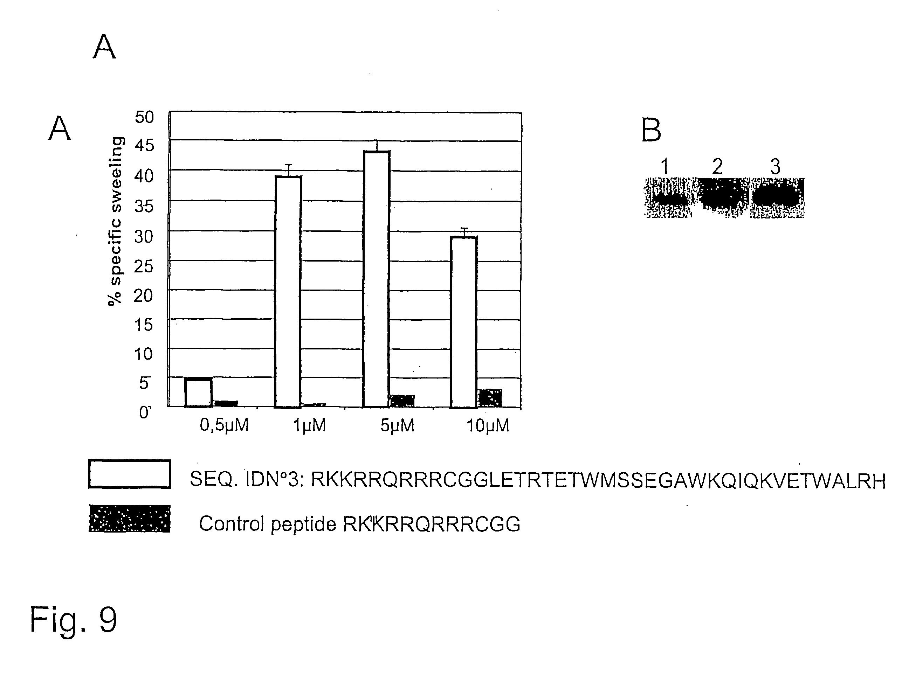

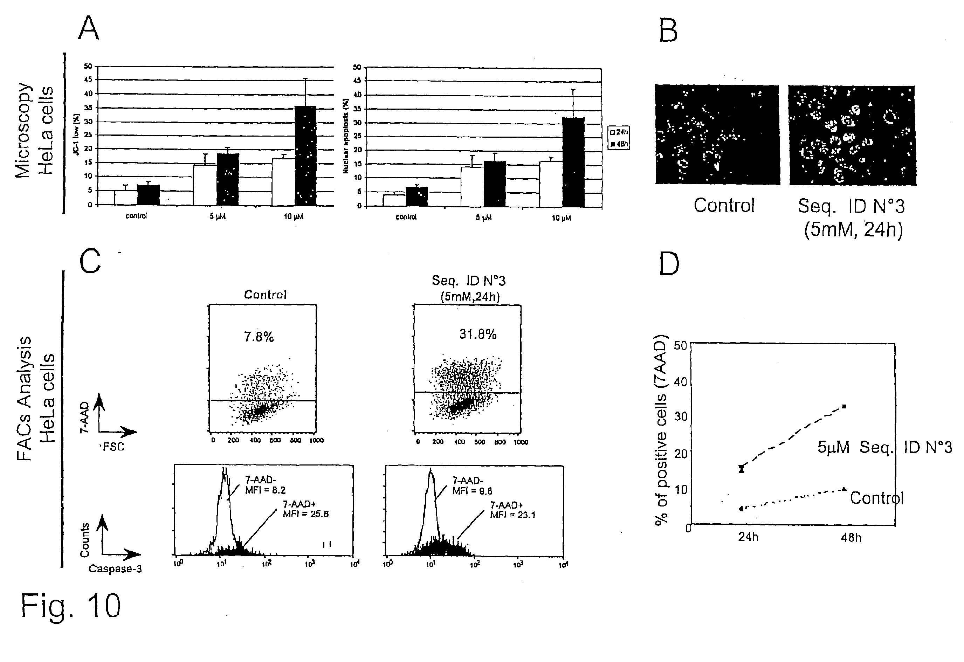

Characterization of apoptosis induced on cultured human cells by treatment with MMP inducing peptides. The example shown relates to peptide seq. ID n°3 RKKRRQRRRCGGLETRTETWMSSEGAWKQIQKVETWALRH. Figure 9 shows the characterization of the peptide on isolated mitochondria, demonstrating dose-dependant specific swelling (A) and release of Cytochrome C (B). The peptide was then tested on cell : the HeLa cells in exponential growth phase were treated with peptide or mock treated, then analyzed by fluorescent microscopy or FACS to ascertain apoptosis. Results shown in figure 10 demonstrate induction of apoptosis in a dose and time related effect.

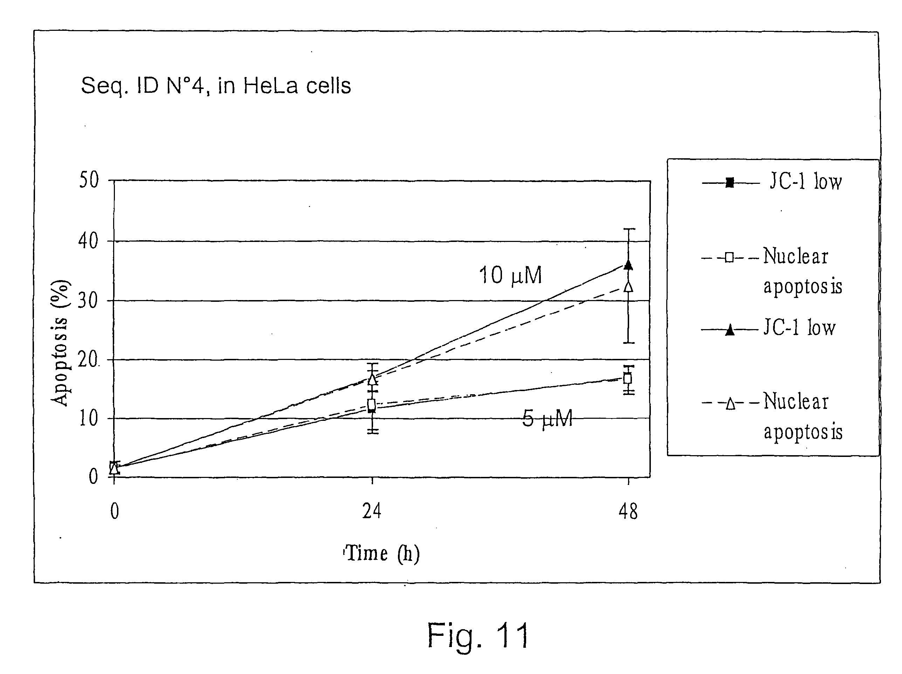

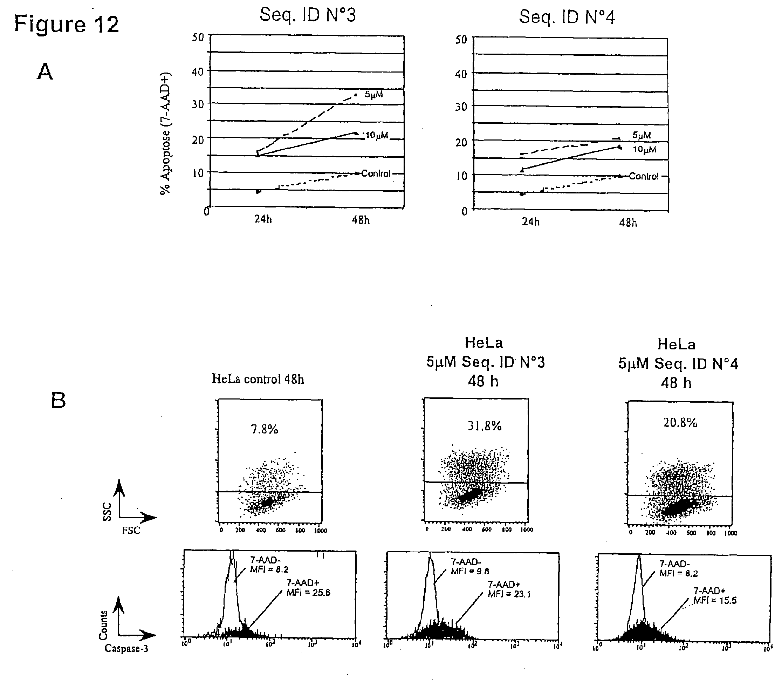

Experiments were performed similarly for peptide SEQ. ID n°4, RKKRRQRRRCGGLANKKGAWLDSTKATRYLVKTESWILRN and are described in Figures 11 and 12. These figure demonstrate *in cellula* mitochondrial destabilization by the peptides.

### Example 6: Induction of cellular apoptosis by MMP inducers on adenocarcinoma cells

Cells in exponential growth phase were cultivated in the absence (Control) or presence of peptides (peptide corresponding to SEQ ID N°11 or related control peptide (not active on mitochondria as ascertained by the real time test) having HWSYWLRPG-GGGGS SEQ ID N°59. Tested human cells where non-adenocarcinoma cells Jurkat and mammary adenocarcinoma MDA-MD-231 cells. 24 hours after incubation with 5 to 20 µM peptide, cells were subjected to FACS analysis to ascertain mitochondrial apoptosis as evidenced by induction of ΔΨm loss and cell membrane permeability induction revealed by FACS analysis. Table 2 show a typical result, expressed as the percent of cells demonstrating evidence of apoptosis, clearly showing induction of apoptosis especially in adenocarcinoma cells.

**Table 2: induction of apoptosis in cultured cells by incubation with mitochondrial permeability inducing peptide. % of apoptotic cells are indicated.**

| | Jurkat cells | Adenocarcinoma MDA-MD231 cells |
|---|---|---|
| Control | <5 | <5 |
| **EHWSYWLRPG-GGGGS** | 8 | 7 |
| Seq. ID n°11 | 18 | 92 |

### Example 7: Induction of cellular apoptosis in HUVEC by MMP inducers.

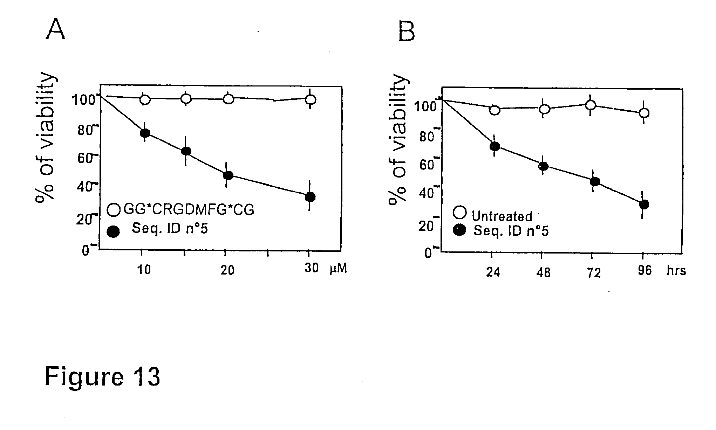

Human umbilical vein endothelial cells were treated with Seq. ID N°5 or relevant control GG*CRGDMFG*CG (not found inducing MMP ion isolated mitochondria), and apoptosis was assessed in a dose and time related manner by FACS analysis. Figure 13 shows the results, clearly demonstrating and apoptosis induction in HUVEC cells treated with the peptide.

### MATERIAL AND METHODS

### Mitochondria isolation and purification

Liver mitochondria were isolated from 3-4 weeks old Balb-c mice (IFFA CREDO, Saint Germain sur l'Arbresle, France). Animals were fasted overnight before being killed by cerebral dislocation. Liver mitochondria were prepared as described (Jacotot, 2000). All steps were carried out at 4°C. Finally, the organelles were resuspended in 'swelling buffer', composed by 200 mM sucrose, 5 mM succinate, 10 mM Tris-MOPS (pH 7.4), 1 mM H₃PO₄, 2 µM Rotenone, and 10 µM EGTA at pH=7.4. Spectrophotometric and flow cytometric analyses were performed in this buffer. The protein content of isolated mitochondria was determined by the Bradford assay.

### Reagents tested on mitochondria

Mitochondrial Membrane Permeabilization (MMP) was induced by 1-30 µM Calcium (CaCl₂, MERCK), and 5 µg/ml Alamethicin (SIGMA). Ruthenium Red (SIGMA) and the PTP inhibitor Cyclosporine A (CSA, BIOMOL) were added at 10 µM 4 minutes before the addition of the MMP-inducing agents. The uncoupler carbonyl cyanide *m*-chlorophenylhydrazone (mClCCP, SIGMA) was used at 20 µM to completely collapse ΔΨm.

### Detection of large amplitude swelling by spectrophotometry

Large amplitude swelling was determined with Ultrospec 3300 pro spectrofluorometer (Amersham Pharmacia Bioech, Cambridge, England) by measuring Absorbance at 545 nm (A₅₄₅) (Bernardi et al., 1992). Mitochondria were resuspended in 1 ml swelling buffer at a protein concentration of 100 µg/ml. Basal absorbance was recorded for 4 minutes, MPP-inducing reagents were added to samples and large amplitude swelling was monitored by the decrease in absorbance for the following 16 minutes. Data were recorded using the Data capture software (Excel, Uppsala, Sweden). Swelling curves were drawn using the Microsoft Excel Software. The positive control of swelling was induced by a 30 µM CaCl₂ treatment. Percentages of specific swelling were calculated as follows: (A _{Ca2+} - A_{Reagent} )x 100 / (A _{Ca2+} - Aᵢₙᵢₜ), were A _{Ca2+} , A_{Reagent} and Aᵢₙᵢₜ correspond to the absorbance value obtained for CaCl₂- treated, reagent - treated and pre-treated mitochondria respectively.

### Flow cytometric analysis of purified mitochondria.

Fluorescence-activated flow cytometry was performed using a 3-color FACSCalibur cytometer equipped with a 15 mW air-cooled 488 nm argon laser (Becton Dickinson, San Jose, CA). Mitochondria analyses were performed after appropriate settings of the Forward angle light scatter (FSC) - Side angle light scatter (SSC) detectors (see Fig. 1). Discrimination between mitochondria and cellular debris and noise background was performed by an appropriate gating on FSC-SSC scatter plots (Fig. 1)(Radcliff G, Jaroszeski MJ 1998 Methods Mol Biol 91:1-24 Basics of flow cytometry). Systematic confirmation of the validity of this gating was obtained by a labeling of mitochondria with the ΔΨm-insensitive mitochondrial dye MitoTracker^{®} Green (MTG, Molecular Probes, Eugene, OR) (Jacotot et al., 2001). Briefly mitochondria (1.5 µg proteins) were respuspended in 400 µl swelling buffer containing 200 nM MTG for 15 min at room temperature. The organelles were immediately analyzed on the cytometer with the CellQuest Pro software (Becton Dickinson). MTG fluorescence was collected in the FL-1 channel (530 +/- 15 nm) and analyzed in logarithmic amplifier mode, using the PMT at a voltage adjusted to 907. Data from 20,000 gated mitochondria were recorded and analyzed.

### Fixed- and real-time analysis of the mitochondrial morphological status using flow cytometry

Mitochondrial size was analyzed through the light scattered by the organelles at low angle using the Forward scatter (FSC) photodiode. Signals were collected using the E-00 setting, with a logarithmic amplification gain of 5.41. Mitochondrial structure was evaluated by the light scattered in the perpendicular direction. It was assessed by the sensitive Side scatter (SSC) photomultiplier tube (PMT), using a voltage of 581, and a linear amplification gain adjusted to 4.27. The morphological changes related to swelling of purified mitochondria were monitored by the increase in mitochondrial size (FSC) and the decrease in the organelle structure (SSC) using the CellQuest Pro software. The FSC/SSC ratio was calculated by the FCS Assistant software and permitted to integrate both variations in size and structure into only one parameter.

FSC is mainly determined by gross mitochondrial size. According to proposed models to calculate light scatter properties (derived from the Rayleigh-Debye-Gans approximation) one can suggest that changes in the refractive index of the mitochondrial matrix also affect FSC.

Fixed-time analyses were performed on mitochondrial samples containing 10 µg proteins in 100 µl of swelling buffer. Samples were submitted to MPP-inducing reagents for 30 min at 25°C. Samples were immediately analyzed by flow cytometry, and data from 20.000 mitochondria were recorded. The percentage of specific swelling determined on forward scatter was calculated as follows;
(FSC_{Ca2+} - FSC_{Reagent} ) x 100 / (FSC_{Ca2+} - FSC_{Co}), were FSC_{Ca2+}, FSC_{Reagent} and FSC_{Co} correspond to the FSC values obtained for CaCl₂- treated, reagent - treated and untreated mitochondria respectively. Similar calculation was used to determine the percentage of specific swelling on SSC and FSC/SSC ratio values.

Real-time experiments were performed on mitochondrial samples containing 1.5 µg proteins in 800 µl (= 2 ng / □l) of swelling buffer. Samples were acquired on the flow cytometer for 5 minutes to record basal morphological criteria. Then reagents were added to samples, and variations in light scattering were recorded for the following 15 minutes. The sample flow rate was settled to 12 µl +/- 3 µl / min for both fixed- and real-time analyses.

Swelling curves were obtained after plotting FSC or SSC parameters versus time, and division of the time scale into discrete periods of 1 minute. The mean FSC and SSC of mitochondria from every period were calculated by the CellQuest Pro software, and were exported into the Microsoft Excel software.

### Fixed- and real-time analysis of ΔΨm in isolated mitochondria using flow cytometry

Mitochondrial transmembrane potential (ΔΨm) was assessed by 5,5',6,6',-tetracholoro-1,1,3,3'-tetraethylbenzimidazolyl carbocyanine iodide (JC-1, Molecular Probes) incorporation. This potential-sensitive cationic dye accumulates in the mitochondrial matrix according to the Nernst equation (Reers et al., 1991). JC-1 green fluorescence, associated with JC-1 monomers was collected in the FL-1 channel. JC-1 orange fluorescence, associated with the formation of J-aggregates was collected in the FL-2 channel (585 +/- 21 nm) (Reers et al, 1991). Both fluorescences were recorded in logarithmic amplifier mode, using the PMT at a voltage adjusted to 907 and 622 respectively. Spectral overlap was avoided by adjusting compensation network as follows: FL1 - 18.5% FL2 and FL2 - 25.4% FL1. The JC-1 orange green fluorescence ratio was calculated with the FCS Assistant software.

Fixed-time detection of ΔΨM was performed on mitochondrial samples containing 10 µg proteins in 100 µl of swelling buffer. Samples were submitted to MPT-inducing drugs for 15 min at 25°C, and JC-1 was added at 800 nM for 15 min at 37°C in the dark. Samples were immediately analyzed by flow cytometry, and data from 20.000 mitochondria (gated on FSC-SSC parameters) were recorded. This assay permitted to quantify the percentage of highly energized mitochondria (with both orange and green fluorescence) and depolarized mitochondria (with green fluorescence only) as depicted in part C2 of Fig.1.

Real-time monitoring of ΔΨm was performed on mitochondrial samples containing 1.5 µg proteins in 400 µl of swelling buffer. JC-1 loading was performed at 400 nM in for 15 minutes at 37°C. Then 400 µl of swelling buffer were added, and samples were acquired on the flow cytometer for 4 minutes to register basal ΔΨm. Then drugs were added to the sample, and ΔΨm variations were recorded for the following 16 minutes. Depolarization curves were drawn as previously indicated for swelling curves; the time scale was divided into discrete periods of 1 minute and the mean of JC-1 orange and JC-1 green fluorescence of the corresponding mitochondrial subsets was calculated by the CellQuest Pro software and exported into the Microsoft Excel software.

### Electron microscopy

Mitochondria were fixed in 3.2 % glutaraldehyde (Sigma, St Quentin Fallavier, France) in 0.1 M Soerensen buffer (0.1 M phosphate, pH= 7.2) (Prolabo, Fontenay sous Bois, France), for 16 hours at 4°C. Then, mitochondria were washed one time in 0.1 M Soerensen buffer pH= 7.2 and two times in 0.1 M cacodylate buffer pH = 7.2 (Prolabo). Samples were fixed in 0.1 M cacodylate buffer containing 1 % OSO₄ acid (Ubichem) for 1 hour at room temperature. After three washings in 1 % OSO₄ acid (Ubichem) in 0.2 M cacodylate buffer, mitochondria were dehydrated in 30 % methanol, stained by 2 % uranyle acetate (Merck, Fontenay sous Bois, France) in 30 % methanol, and dehydrated in a series of ethanol solutions (50-100 %) . Then samples were incubated in propylene oxyde for 15 min, and embedded in epoxy (Epon 812, Merck). After sectioning by a Leica ultramicrotome, samples were stained by uranyle acetate and Pb-citrate (Merck). Observations were performed using a Jeol 1200EX microscope.

### Detection of Caspase-3 activation and apoptosis

Caspase-3 activation was performed using a fluorescent inhibitor of caspase-3 (FLICA, FAM-DEVD-FMK) from the CaspaTag TM kit (Intergen). The loss of plasma membrane integrity occurring during apoptosis of HeLa cells was determined by 7-Amino Actinomycin D (7-AAD) incorporation (SIGMA). 20 mg/ml 7-AAD was added to cells at 37°C for 15 min. Cells were trypsinized, resuspended in PBS and immediately acquired on the FACSCalibur. FAM-DEVD-FMK related fluorescence was detected using the Fl-1 channel. 7-AAD fluorescence was collected in the Fl-3 channel (>650nm).

### Apoptosis detection by fluorescence microscopy

HeLa cells were stained by 1 µM and Hoechst 33-342 JC-1 for 15 minutes and observe d using a DM IRB inverted fluorescence microscope (Leica).

### Statistical analysis

Correlations were calculated by linear regression analysis. a p value < 0.05 was considered as significant. For each analysis, R² is indicated.

### Caption of figures

### Fig. 1: Cytofluorometric identification of isolated mitochondria.

A. Ultrastructural observation of purified mouse liver mitochondria. A representative electron micrograph is shown. **B.** Flow cytometric determination of size (Forward Scatter, FSC) and granularity (Side Scatter, SSC) of liver mitochondria. Appropriate settings of FSC (logarithmic scale) and SSC (linear scale) permit to detect events with low SSC and variable FSC (region 1), and events scattered with a variable SSC and homogenous FSC (ellipsoid gate, region 2). **C.** Mitochondria content of each region. Mitochondrial staining with the ΔΨm -insensitive mitochondrial dye MitoTracker^{®} Green (MTG , 200 nM, black histogram) in parallel to unstained samples (Co. grey line). The percentage of labeled mitochondria (MTG+ events) is indicated for each selected region (MTG). **D.** Evaluation of ΔΨm status in purified mitochondria. Isolated mitochondria were submitted 15 min. to the ΔΨm -sensitive fluorochrome JC-1 (800 nM) and subjected to flow cytometric determination of the fluorescence in both e FL-1 and FL-2 channels. The percentage of events is indicated in each corresponding quadrants. Events located in the region 1 (defined in B.) remained unstained by MTG and JC-1 and corresponded noise background, whereas region 2 (defined in B.) corresponded to 95.5% mitochondria with a highly energetized state (90.5% JC-1 orange mitochondria).

### Figure 2: Dose-response evaluation of mitochondrial swelling by fixed-time flow cytometry.

Ca2+ was added to purified mitochondria at the indicated doses. **A.** Ca2+ induces dose-dependent SSC decrease and FSC increase (The mean value of FSC and SSC mean are indicated in scatter plots). Note that Ca²⁺-treated mitochondrial population harbors a characteristic 'croissant-like' shape in the right and inferior part of FSC-SSC scatter plots. **B.** Comparative analysis of Ca²⁺ -induced mitochondrial swelling analyzed by spectrophotometry and flow cytometry (FSC and SSC parameters). Mitochondria were treated 30 min. with the indicated doses of Ca in the presence or absence of 10 µM CsA. **C.** Comparative analysis of absorbance at 545nm versus FSC/SSC ratio. **D.** Linear correlation between Δ_{FSc} and □A₅₄₅. □A₅₄₅, corresponds to the difference between the absorbance of the untreated sample and the Ca²⁺ -treated sample after 30 min of monitoring. Δ_{FSc} and Δ_{SSC} correspond to the similar calculation for FSC and SSC parameters. **E.** Linear correlation between Δ_{SSC} and ΔA₅₄₅. Parts C and D indicate that mitochondrial swelling evaluated by both FSC increase and SSC decrease was significantly correlated with decrease in absorbance at 545nm.

### Figure 3: Fixed-time versus Real-time detection of Ca²⁺-induced swelling by flow cytometry.

**A,** C: Fixed-time analysis of mitochondria after 20 min incubation in the absence (A) or presence (C) of 30 µM Ca²⁺. Representative dot-plot (SSC/FSC) analysis and electron micrographs are shown. **B, D:** Real-time monitoring of FSC (left), SSC (middle), and FSC/SSC ratio (right) absence (B) or presence (D) of 30 µM Ca²⁺. Basal level of FSC, SSC, and FSC/SSC ratio were recorded for 5 minutes. Then, 30 µM calcium were added (vertical arrow) and morphological variations were monitored for the following 15 minutes. The white line corresponds to the mean FSC, SSC and FSC/SSC values. Under each real-time dot-plot, the velocity of mitochondrial modifications is calculated (Δ_{FSC}/min +/- SD, Δ_{SSC}/min +/- SD, and Δ_{FSC/SSC}/min +/- SD; n=7).

### Figure 4: cytofluorometric real-time detection of PTP-dependant and PTP-independent mitochondrial swelling.

(A and B) Time-course of mitochondrial swelling monitored by flow cytometry and spectrophometry. Control mitochondria, and samples treated with 20 µM mClCCP, 30 µM Ca²⁺, and 5 µM alamethicin. The CsA inhibition experiment was performed by pre-treating mitochondria by 10 µM CsA for 5 minutes prior to Ca²⁺ addition. Arrows indicate addition of the corresponding molecule. A. Mean FSC (black curve) and SSC (grey curves) values are recorded for 20 min. **B**. measurement of absorbance at 545nm by spectrophometry (classical swelling assay). **C.** Real-time monitoring of mitochondrial swelling induced by 30 µM Ca²⁺, assessed by the FSC/SSC ratio (black line) versus absorbance at 545 nm (grey line). **D.** velocity of FSC/SSC ratio modification (Black line, grey histogram) versus absorbance at 545 nm (grey line). Means and standard deviation were obtained on 7 independent experiments.

### Figure 5: Real time incorporation of JC-1 in isolated mitochondria.

Basal autofluorescence of mitochondria were recorded for 2 min. Then, mitochondria were exposed to the ΔΨm-sensitive mitochondrial dye JC-1 (400 nM) followed by cytofluorometric two-color analysis for 23 minutes. **A.** Time-dependant incorporation of JC-1.White curves correspond to the mean of JC-1 green (upper panel; FL-1) and orange fluorescence (lower panel; FL-2). Note that JC-1 orange fluorescence (J-aggregates formation) reach a plateau after 15-20 min incubation. **B.** Dot-plot FL-1/FL-2 analysis of JC-1 incorporation. Recorded events (A.) were re-analyzed as the sum of events during the following time-periods; 0-2min (basal auto fluorescence), 2-8 min, 8-14 min, 14-20 min and 20-25 min. An appropriate quadrant permits to quantify the percentage of unstained, single stained, and double stained mitochondria.

### Figure 6: Real-time monitoring of ΔΨm by flow cytometry.

Real-time co-monitoring of JC-1 orange fluorescence (**A.** and **B.**), JC-1 green fluorescence (**C.** and **D.**) and FSC/SSC ratio (**E.** and **F.**). JC-1 loaded mitochondria were exposed or not (Co.) to 20 µM mClCCP, 30 µM Ca²⁺, or 5 µM Alamethicin. Time-F1-2 (**A.**) and Time-Fl-1 (**C.**) dot-plots are shown. Arrows indicate the moment of drugs addition. Differences between final and initial mean values of fluorescence are indicated (Δ). Depolarization curves (**B.**, **D.**, and **F.**) are derived from corresponding dot-plot (mean of events on a per-minute basis) and expressed in linear scales.

### Figure 7: Co-monitoring of SSC and ΔΨm by Real-time flow cytometry in Ca²⁺ treated mitochondria.

Purified mitochondria were exposed to 30 □M Ca²⁺ (**A.**) or 20 µM mClCCP (**B.**) followed by SSC/FSC fixed-time dot-plot analysis (1) and real-time FCM (**2**, **3**, **4**, **5**). **1.** Mitochondria were analyzed before (to) and after 20 min treatment with Ca²⁺ or mClCCP. The ellipsoid region (red dots) corresponds to intact mitochondria and orange dots (all events outside the region) correspond to swollen mitochondria. **2.** Time-SSC dot-plot analysis. **3.** Time-FL-2 (JC-1 orange fluorescence) dot-plot analysis. Note that after addition of Ca²⁺ (arrows in A.), mitochondria remained red (i.e. inside the ellipsoid region defined in 1) for a few second, and rapidly became orange simultaneously to internal structure modification and a ΔΨm collapse. Stabilization was achieved 7 minutes after Ca²⁺ addition. **4.** Comparative mean SSC and mean ΔΨm (JC-1 orange fluorescence) time-response curves (n=7 +/-SD). **5.** Correlation between SSC and ΔΨm variations.

### Figure 8: FCM analysis of PTP-dependant and PTP-independent mitochondrial membrane permeabilization.

Mitochondria were exposed 5 minutes to 50 µM BA, 10 µM Csa, or 10 µM Ruthenium red followed by 30 min treatment with Ca²⁺ (30 µM) or alamethicin (5 µg/ml). Then, mitochondria were subjected to FCM analysis of ΔΨm (JC-1 orange fluorescence) and SSC (Mean +/-SD; n=3).

### Fig.9. The peptidic SEQ ID N°3 induces MMP.

**A.** Activity was evaluated on isolated mitochondrial and demonstrated specific swelling, as opposed to the control peptide. **B.** Cytochrome C release: untreated mitochondria (1), or treated with 5 µM Alamethicin (2) or 10µM peptide seq. ID N°2 during 30 minutes were assayed for cytochrome C release by western blot on the supernatant.

### Figure 10: Cellular apoptosis induced by MMP modulating peptides.

HeLa cells were incubated for 24 or 48h with 5 or 10 µM of peptide Seq. ID n°3 or left untreated. **A.** Cells were then stained with JC-1 and Hoechst dyes. Observation and counting of JC-1 low cells and nuclear condensation were done by immunofluorescence microscopy (mean of 3 experiments). **B.** Fluorescence microscopy of the cells, after 24 hours incubation with or without 5 µM peptide and staining with JC-1 dye. **C.** Same conditions than B, and staining with 7AAD and detection of Caspase 3 activity using FACS analysis, **D.** FACS analysis of 7AAD staining.

### Fig 11. Seq ID n°4 exhibits mitochondrio-toxic activity.

Peptidic sequence was added at 5 or 10 µM on HeLa cells and apoptosis was characterized by FAX at 24 and 48 hours.

### Fig 12. Compared cytotoxic effects of Seq. ID n°3 and 4.

HeLa cells were incubated for 24 or 48h with 5 or 10 µM of peptide Seq. ID n°3 or Seq ID n°4 or left untreated. **A.** FACS analysis of 7AAD staining. **B.** 24 hours incubation with or without 5 µM peptides and staining with 7AAD and detection of Caspase 3 activity using FACS analysis.

### Fig 13. Cytotoxic effects of Seq. ID n°5 on Human endothelial cell (HUVEC).

HUVEC cells were incubated for 24 to 96h with 5 or 30 µM of peptide Seq. ID n°5, control peptide, or left untreated. Apoptosis was characterized by FACS analysis after 7AAD staining. **A.** Dose- response versus control peptide. **B.** Increasing incubation times with or without 10 µM peptides and staining with 7AAD and detection by FACS analysis.

## Claims

1. A method for screening modulators of mitochondrial function comprising adding a compound to be tested in a purified, isolated mitochondria preparation and simultaneously using fluorimetric analysis of mitochondrial morphology, and especially real-time fluorimetric analysis, combining analysis of morphometric parameters (SSC/FSC parameters) with analysis of membrane integrity by dye fluorescence.

2. The method of claim 1, wherein the analysis of the membrane integrity is performed using the JC-1 dye to characterize mitochondrial transmembrane potential Δψm and study any change of the mitochondrial membrane permeability.

3. The method of claim 1 or 2, wherein the mechanism of mitochondrial membrane permeabilization is further **characterized** using pharmacological inducers or modulators of mitochondrial permeability such as Calcium, CCCP, Alamethicin, Bongkrekic acid, ruthenium red, cyclosporin A, DIDS.

4. The method of anyone of claims 1 to 3, further comprising studying endogenous or xenogenic mitochondrial regulators such as Bcl-2 family members and respiratory inhibitors on PTP-related MMP.

5. The method of anyone of claims 1 to 3, further comprising identifying compounds inducing MMP or preventing MMP induced by other agents, alone or in combination.

6. The method of anyone of claims 1 to 5, further comprising designing and defining new agents aiming at modulating MMP.

7. The method of anyone of claims 1 to 6, further comprising defining and manufacturing reagents or kits of reagent to put the method in use.

8. The method according to anyone of claims 1 to 4, further comprising characterizing mitochondrial reactivity in specific mitochondria, to be used to ascertain mitochondrial function in mitochondria from various tissues of physiological or pathological status, or pathology sources such as tumors.

9. The method according to anyone of claims 1 to 4, further comprising the diagnosis or characterization of mitochondrial function in patients with diseases, and especially genetic diseases or metabolic diseases.

10. Agents identified by using method according to claims 1 to 4, and shown to be able to modulate MMP on isolated mitochondria.

11. Peptides according to claim 10, having MMP modulating potency, selected in the group comprising:
| | |
|---|---|
| ATLSALLAALRRIQRA | (SEQ ID N°1) |
| RKKRRQRRRGGATLSALLAALRRIQRA | (SEQ ID N°2) |
| RKKRRQRRRCGGLETRTETWMSSEGAWKQIQKVETWALRH | (SEQ ID N°3) |
| RKKRRQRRRCGGLANKKGAWLDSTKATRYLVKTESWILRN | (SEQ ID N°4) |
| GG*CRGDMFG*CGGLLFIHFRIGSRHSRIG | (SEQ ID N°5) |
| RIEIWILRH | (SEQ ID N°6) |
| RIAIWILRH | (SEQ ID N°7) |
| RKKRRQRRRGGRIEIWILRH | (SEQ ID N°8) |
| RKKRRQRRRGGRIAIWILRH | (SEQ ID N°9) |
| EHWSYWLRPGGGLLFIHFRIGCRHSRIG | (SEQ ID N°10) |
| EHWSYWLRPGGGGGSLLFIHFRIGCRHSRIG | (SEQ ID N°11) |
and their derivative,
said peptides advantageously comprising in the C-terminal and N terminal position a stabilizing group such as an amide alkyl or acyl group and a marker or linking group, such as the biotinyl group, and optionally including L- or D- aminoacids retro-inverso, reduced peptidic backbone, or being translated into pseudo peptides,
said peptides being also optionally bound to a peptidic delivery system, and optionally comprising a linker between said peptides and delivery system, the peptidic delivery system advantageously being an extra cellular or intracellular targeting sequence, an antibody or a fragment thereof (ScFv) and the linker can be a sequence allowing the peptide to adopt an helical structure independently of the peptidic delivery system, and can be formed by 2 to 6 aminoacids such as alanine or glycine, or a disulfide bridge.

12. Peptides obtained by anology to peptides described in claim 11, presenting features characteristics of a MMP modulating function, defined as:
- containing at least 8 amino acids, and up to 50 amino acids
- at least a part of the peptide structure is an amphipathic alpha helix,
- 2, 3 or 4 amino acids are positively charged (lysine [K] or arginine [R]) and are located on the same side of the helix,
- when the helix is projected using helical wheel representation, the R and/or K amino acids form a cluster (see figure 15),
- when added to purified, isolated, mitochondria they induce changes (ultrastructural or membrane permeability).
Example of such peptides are selected below :
| | |
|---|---|
| RKKRRQRRRGGGAWKHAQRIEIWILRH | (SEQ ID N°12) |
| RKKRRQRRRGGGAWKHAQRIETWILRH | (SEQ ID N°13) |
| RKKRRQRRRGGGAWKHAQRVESWILRN | (SEQ ID N°14) |
| RKKRRQRRRGGGAWKRACRMETWILRH | (SEQ ID N°15) |
| RKKRRQRRRGGGAWKQIQKVETWALRH | (SEQ ID N°16) |
| RKKRRQRRRGGGAWRQVEKVETWALRH | (SEQ ID N°17) |
| RKKRRQRRRGGGAWKHAQRIAIWILRH | (SEQ ID N°18) |
| AWKHAQRIAIWILRH | (SEQ ID N°19) |
| GG*CRGDMFG*CGGRIEIWILRH | (SEQ ID N°20) |
| GG*CRGDMFG*CGGRIAIWILRH | (SEQ ID N°21) |
| GG*CGRGDSPG*CGGRIEIWILRH | (SEQ ID N°22) |
| GG*CGRGDSPG*CGGRIAIWILRH | (SEQ ID N°23) |
Where *C = cysteine engaged in cycling disulfide bridge
| | |
|---|---|
| EHWSYWLRPGGGRIEIWILRH | (SEQ ID N°24) |
| EHWSYWLRPGGGRIAIWILRH | (SEQ ID N°25) |
| EHWSYWLRPGGGGGSRIEIWILRH | (SEQ ID N°26) |
| EHWSYWLRPGGGGGSRIAIWILRH | (SEQ ID N°27) |
| EHWSYWLRPGGGGGSGAWKHAQRIEIWILRH | (SEQ ID N°28) |
| EHWSYWLRPGGGGGSGAWKHAQRIAIWILRH | (SEQ ID N°29) |
| EHWSYWLRPGGGLLFIHFKIGCKHSKIG | (SEQ ID N°30) |
| EHWSYWLRPGGGGGSLLFIHFKIGCKHSKIG | (SEQ ID N°31) |
| EHWSYWLRPGGGLLFIHFRIGSRHSRIG | (SEQ ID N°32) |
| EHWSYWLRPGGGGGSLLFIHFRIGSRHSRIG | (SEQ ID N°33) |
| EHWSYWLRPGGGLLFIHFKIGSKHSKIG | (SEQ ID N° 34) |
| EHWSYWLRPGGGGGSLLFIHFKIGSKHSKIG . | (SEQ ID N°35) |
in which the W6 residue can be replaced by the D-aminoacid _{d}w. These peptide are claimed along with their derivatives as dezfined in claim 10.
